# EUROPEAN PATENT SPECIFICATION

(11) **EP 3 065 578 B1**
(45) Date of publication and mention of the grant of the patent: **26.12.2018**
(21) Application number: 14802594.3
(22) Date of filing: 10.11.2014
(51) Int. Cl.: A24B 13/00, A24B 15/12, A24B 15/16, A24B 15/28, A24B 15/30, A24B 15/42, A61Q 11/02, A61K 31/047, A61K 33/16, A23G 4/06, A23G 4/10, A61Q 11/00, A61K 8/97

(54) **ORAL SMOKELESS TOBACCO PRODUCTS AND ORAL SMOKELESS NON-TOBACCO SNUFF PRODUCTS**
ORALE RAUCHFREIE TABAKPRODUKTE UND ORALE RAUCHLOSE TABAKFREIE SCHNUPFTABAKPRODUKTE MIT XYLIT UND OPTIONAL FLUORID
PRODUITS DE TABAC SANS FUMÉE À USAGE ORAL ET PRODUITS À PRISER SANS TABAC SANS FUMÉE À USAGE ORAL AVEC XYLITE ET OPTIONALLEMENT FLUORURE

(30) Priority: 08.11.2013 EP 13192179
(43) Date of publication of application: 14.09.2016
(73) Proprietor: Swedish Match North Europe AB, 118 85 Stockholm (SE)
(72) Inventor: KINDVALL, Mårten, S-415 24 Göteborg (SE); WERDINIUS, Christian, S-417 20 Göteborg (SE)
(74) Representative: Valea AB
(86) International application number: PCT/EP2014/003003
(87) International publication number: WO 2015/067372

(56) References cited:
- WO-A1-99/13870
- WO-A1-2009/073190
- GB-A- 2 000 674
- US-A- 4 284 650
- US-A1- 2013 152 953
- D. Kandelman ET AL: "A 24-month Clinical Study of the Incidence and Progression of Dental Caries in Relation to Consumption of Chewing Gum Containing Xylitol in School Preventive Programs", Journal of Dental Research, vol. 69, no. 11 1 November 1990 (1990-11-01), pages 1771-1775, XP055162395, Retrieved from the Internet: URL:http://jdr.sagepub.com/content/69/11/1 771.abstract [retrieved on 2015-01-15]
- K K Makinen ET AL: "Xylitol Chewing Gums and Caries Rates: A 40-month Cohort Study Introduction", J Dent Res, 1 December 1995 (1995-12-01), pages 1904-1913, XP055162380, Retrieved from the Internet: URL:http://deepblue.lib.umich.edu/bitstrea m/handle/2027.42/68231/10.1177_00220345950 740121501.pdf?sequence=2 [retrieved on 2015-01-15]
- "Guidelines on Xylitol Use in Caries Prevention", AMERICAN ACADEMY OF PEDIATRIC DENTISTRY CLINICAL GUIDELINES , no. 175 1 January 2011 (2011-01-01), XP055162381, Retrieved from the Internet: URL:http://www.aapd.org/media/Policies_Gui delines/G_XylitolUse.pdf [retrieved on 2015-01-15]
- Anonymous: "Smokey Mountain Snuff - FAQ", , 25 November 2012 (2012-11-25), XP055316246, Retrieved from the Internet: URL:https://web.archive.org/web/2012112501 5327/http://www.smokeysnuff.com/faq.html [retrieved on 2016-11-03]
- COX S D ET AL: "A new mechanism of action of fluoride on streptococci", BIOCHIMICA ET BIOPHYSICA ACTA (BBA) - GENERAL SUBJ, ELSEVIER, AMSTERDAM, NL, vol. 1428, no. 2-3, 5 August 1999 (1999-08-05), pages 415-423, XP004276464, ISSN: 0304-4165, DOI: 10.1016/S0304-4165(99)00052-5

## Description

### Technical field

The present document relates to oral smokeless tobacco products and oral smokeless non-tobacco snuff products comprising xylitol.

### Background

Certain groups of facultative anaerobic microorganisms present on the surface of the teeth, called dental plaque, present in the form of a bio-film, are capable of metabolizing (fermenting) carbohydrates into organic acids, especially lactic acid. Representative types of facultative anaerobic microorganisms present in the oral cavity, in saliva and dental plaque, are mutans streptococci (MS) (including both *S*. *mutans* and *S*. *sobrinus* species) and lactobacilli. As a consequence of the metabolism of carbohydrates into organic acids, plaque-pH is lowered from the natural pH of saliva of 6.5 -7.5 down to pH 4-5 where calcium and phosphate ions are dissolved. When the saliva is neutralized to normal (natural) pH in the oral cavity, calcium and phosphate ions in the saliva are precipitating on the tooth surfaces and the degraded areas are regenerated in a "remineralization" process. Below a pH of 5.5 there is a net demineralization of enamel and dentine where calcium and phosphate are dissolved faster than they are regenerated and caries lesions will eventually occur. Consequently, frequent exposure to carbohydrates, resulting in a low pH in the oral cavity, may with time cause caries lesions. It is well known that the carries risk is lower if demineralization is avoided or if remineralization is stimulated.

Oral smokeless tobacco products are used in the oral cavity in direct contact with oral mucosa, where they are allowed to deliver flavour(s) and biologically active substances. The products are used in the mouth for an extended period of time, normally between 10 and 60 minutes and sometimes even longer. Typical oral smokeless tobacco products are for example moist snuff such as snus, and chewing tobacco, hard snuff and oral dry snuff.

Oral smokeless non-tobacco snuff products are products resembling oral smokeless tobacco products and are used in the same way as oral smokeless tobacco products.

Most oral smokeless tobacco products, as well as oral smokeless non-tobacco snuff products, contain carbohydrates. Tobacco naturally contains sugars and curing of the tobacco increases the sugar content as polysaccharides are broken down to sugar (mono-and disaccharides). Oral smokeless non-tobacco snuff products are usually based on plant materials which normally contain carbohydrates, such as sugars and starch. Oral smokeless tobacco products, as well as oral smokeless non-tobacco products, may also have carbohydrates added to the raw material in the manufacturing process for improving the taste and/or the texture of the product.

Generally, use of oral smokeless tobacco products as well as oral non-tobacco snuff products, with high carbohydrate content, naturally or by way of additives, and/or low pH and/or low buffer capacity, increases the risk of demineralization of the teeth. In addition, intake of dietary carbohydrates, which is the greatest cause of dental caries, significantly increases said demineralization.

Hence, there exists a need for oral smokeless tobacco products and oral smokeless non-tobacco snuff products with an improved ability to counter-act demineralization of the teeth and/or the advent of carries lesions.

Moreover, oral smokeless tobacco products and oral smokeless non-tobacco snuff products may, depending on the components used, be associated with various production-oriented problems. Consequently, there also exists a need for improved oral smokeless products and oral non-tobacco snuff products that can be efficiently produced. US20130152953A1 discloses smokeless tobacco compositions comprising in the end product 5,23wt% xylitol and 2-6wt% water. The webpage
https://web.archive.org/web/20121125015327/http://www.smokeysnuff.com/faq.html discloses oral tobacco pouches (snus) comprising xylitol. Cox S D et al: A new mechanism of action of fluoride on streptococci discloses the effect of 0,5; 1; 5mM NaF on Streptococci bacteria.

### Summary

It is an object of the present document to solve or at least mitigate the above defined problems in the art. The present document is therefore directed to an oral smokeless tobacco product or an oral smokeless non-tobacco snuff product, comprising xylitol in an amount of from 6-20 w/w % of the final product, such as from about 6-15 w/w % or about 9-9.9 % of the final product. The water content of the final product is about 20 to 60 w/w %, such as 26 to 58 w/w %, and wherein said oral smokeless non-tobacco snuff product comprises non-tobacco plant material, wherein said non-tobacco plant material is non-tobacco fibers.

An oral smokeless non-tobacco snuff product comprises non-tobacco plant materials, such as non-tobacco fibers, such as oat fibres, apple fibres, sugar beet fibres, potato fibres, corn fibres, buckwheat fibres, cocoa fibres, and/or cellulose fibres.

A product according to the present document may further comprise fluoride added as sodium fluoride and/or sodium monofluorophosphate. The fluoride may be present in an amount of from about 0.0050 to about 0.0500 w/w % of the final product, such as from about 0.0150 to about 0.0300 w/w % of the final product.

The oral smokeless tobacco product may be moist snuff, such as snus, or chewing tobacco. The oral smokeless non-tobacco snuff product may be shaped into a form resembling moist snuff, such as snus, or chewing tobacco, oral dry snuff or hard snuff.

A product according to the present document may be provided in form of pouches, or loose in particulate form, packed in cans or boxes. In particular, moist snuff or an oral smokeless non-tobacco snuff product resembling moist snuff may be contained in pouches.

The product may also be used for delivering xylitol to the oral cavity, e.g. the product may be used as a delivery device for delivering xylitol to the oral cavity of a user.

### Brief description of drawings

Figure 1, shows the calculated xylitol amount extracted in the mouth in mg and as percentage of the amount of xylitol present in the unused product respectively.
Figure 2, shows the concentration of fluoride in saliva when using an oral smokeless tobacco product comprising fluoride according to the present document compared to that of prior art products comprising fluoride.
Figure 3, shows the concentration of fluoride in saliva when using an oral smokeless non-tobacco product comprising fluoride according to the present document compared to that of prior art products comprising fluoride.
Figure 4, shows the xylitol concentration in saliva during four hours of normal use of a snus product.
Figure 5, shows the relation between xylitol concentration in snus and saliva.
Figure 6, shows the xylitol concentration in saliva during two hours of continuous use of a snus product.

### Detailed description

The present document provides an oral smokeless tobacco product or an oral smokeless non-tobacco snuff product (herein below also referred to as the "product") by which the above-mentioned problems may be overcome or be significantly improved.

The product has preventing properties towards periodontal disease and/or dental caries and may be more efficiently manufactured. The product comprises xylitol in an amount of 6-20 w/w %, preferably 6-18 w/w %, 6-15 w/w %, 6-11 w/w %, 7-20 w/w %, 7-18 w/w %, 7-15 w/w %, 7-11, 8-20 w/w%, 8-18 w/w %, 8-15 w/w %, 8-11 w/w% or 9-20 w/w % of the final product. That is, the xylitol is present in the product in the amount of at least equal to or any number in between of 6, 7, 8, 9, 10, 11, 12, 13, 14, 15, 16, 17, 18, 19 and 20 w/w %. In yet another form the product may comprise xylitol in an amount of 9.0-9.9 w/w % or 9-11 w/w% of the final product. For some users an amount of xylitol in the product of more than 10 w/w % it may have a laxative effect.

Xylitol in an amount lower than 5 w/w % in the product it is believed to not contribute to any significant clinical effect, i.e., would not show any effect on the content of mutans streptococci in the mouth. It has been found that a product comprising at least 6 w/w % xylitol provides a concentration of xylitol in saliva which enables a sufficient reduction of mutans streptococci in the oral cavity. In order to ensure that a sufficient amount of xylitol is released from a portion of a product (e.g. in the form of a pouch product or a portion of loose snuff), this should contain at least 0.055 g of xylitol, such as 0.06, 0.07, 0.08, 0.09, 0.1, 0.12, or 0.15 g per portion product (i.e. in each unit/portion of the product), provided the w/w % of xylitol does not exceed 20 w/w %. Typically, a product of the present document enables an average concentration of xylitol in the saliva of at least 0.02 w/w %, such as at least 0.03 w/w %, 0.04 w/w % 0.05 w/w%, 0.06 w/w %, 0.07 w/w %, 0.08 w/w %, 0.09 w/w %, 0.1 w/w%, 0.2 w/w% or 0.3 w/w% to be obtained during normal use of a product according to the present document, such as for use during 15-120 min, such as 30-120 min, 15-60 min,15-30 min, 15-45 min or 30-60 min, e.g. 45 min. Xylitol in an amount greater 20 w/w % in the product would cause production problems, such as packaging inefficiency due to e.g. increased stickiness of the product.

The product of the present document may be in form of pouches. A pouch may weigh from about 0.3 to about 1.5 g, such as 0.5 to 1.5 g. A portion of a product according to the present document is typically weighs from about 0.3 to about 1.5 g, such as 0.5 to 1.5 g.

Typically, a product of the present document is used for a period of time of about 10 to 120 min, in particular at least 15 min or 30 min.

In one form the product may comprise xylitol in an amount of 6-15 w/w % of the final product.

Xylitol is a sugar alcohol that can be used as a sugar substitute. Xylitol has the formula (CHOH)₃(CH₂OH)₂ and is an achiral isomer of pentane-1,2,3,4,5-pentol. Xylitol may be used in oral tobacco products e.g., as a sweetener typically in an amount of 1 to 3 w/w % or in nicotine or tobacco containing tablets in an amount of at least 50 w/w%. The sensory characteristics, of especially moist snuff such as snus, are very important for the consumer and therefore, a significant increase of the xylitol amount in such a product would normally be expected to increase the sweetness of the product and consequently the taste of it. In addition, it could also be expected that a significant increase of xylitol, would result in an increased stickiness, which could cause manufacturing problems and even result in a final product that would be to the consumer's dissatisfaction. However, it has surprisingly been shown that addition of xylitol within the range of the present document does not cause any manufacturing problems nor does it change the taste of the final product; on the contrary it does improve the manufacturing process, since the cohesiveness of the product increases and thereby, transportation and packaging of the product is facilitated.

Even though xylitol is known for its caries-preventing properties, all studies made of its use for that purpose in, e.g., chewing gums, mouth wash and toothpaste, show that a much higher daily dosage of xylitol than that according to the present document is needed in order for an improvement to be proved, if at all (cf., P. Milgrom et al., J Dent Res. 2006 February; 85(2): 177-181). The product of the present document has surprisingly been shown to reduce the amount of mutans streptococci (MS) (including both *S*. *mutans* and *S*. *sobrinus* species) in the oral cavity to a lower concentration than it could be expected from studies made in the field of cariology and/or the amount of xylitol in commercially available xylitol containing products. Since the amount of bacteria causing caries is reduced to an unexpected low level, use of the products of the present document consequently may have a much better effect against periodontal disease and/or dental caries and that at significant lower daily dosages of xylitol than that known. In other words, delivery of xylitol in the oral cavity via the product of the present document in form of, e.g., a pouch has been shown to be superior compared to other delivery forms. Also, the product comprising xylitol as defined herein may act as a extended release formulation for the delivery of xylitol to the oral cavity.

The product of the present document may also comprise fluoride. The fluoride may be added in any form. However, sodium fluoride (NaF) or sodium monofluorophosphate (Na₂PO₃F) are preferred. Sodium fluoride may be most preferred, except in cases where native calcium is present in the tobacco material used. In the latter case, sodium monofluorophosphate may be preferred; since there is a risk that sodium fluoride may not be released in the oral cavity due to the presence of calcium in the tobacco material. The content of fluoride in the product, available for release in the oral cavity, as meant herein, is the amount of fluoride present in the fluoride compound, e.g. sodium fluoride or sodium monofluorophosphate, calculated as fluoride w/w % based on the total weight of the final product.

The fluoride may be present in the product in an amount of from 0.0010-0.1000 w/w % (10-1000 ppm) of the final product.

In one form, the product of the present document may comprise fluoride in an amount of 0.0050-0.0500 w/w % (50-500 ppm) of the final product.

In yet another form, the product of the present document may comprise fluoride in an amount of 0.0150-0.0300 w/w % (150-300 ppm) of the final product.

In still yet another form, the product of the present document may comprise fluoride in an amount of 0.0200-0.0250 w/w % (200-250 ppm) of the final product.

Fluoride has a well-documented effect in preventing tooth decay by strengthening the enamel. Topically delivered fluoride has a better anti-caries effect than systemic intake of it. It has been shown that the fluoride level in the saliva and plaque fluid during an acid attack, after e.g. a meal, is critical for inhibiting demineralization and enhancing mineralization (cf., John D. B. Featherstone, Community Dent Oral Epidemiology, 1999 (27), 31-40). However, after tooth brushing in the morning with a fluoride containing dentifrice, the fluoride level in the mouth returns to resting levels after 1-2 h or even faster, if the mouth is rinsed with water or something is eaten afterwards (Bruun C., et al., Community Dent. Oral Epidemiology, 1982 (10), 124-129). Use of a product comprising fluoride as defined herein helps sustaining a therapeutically effective concentration of fluoride in the oral cavity over prolonged periods of time, like extended release formulation.

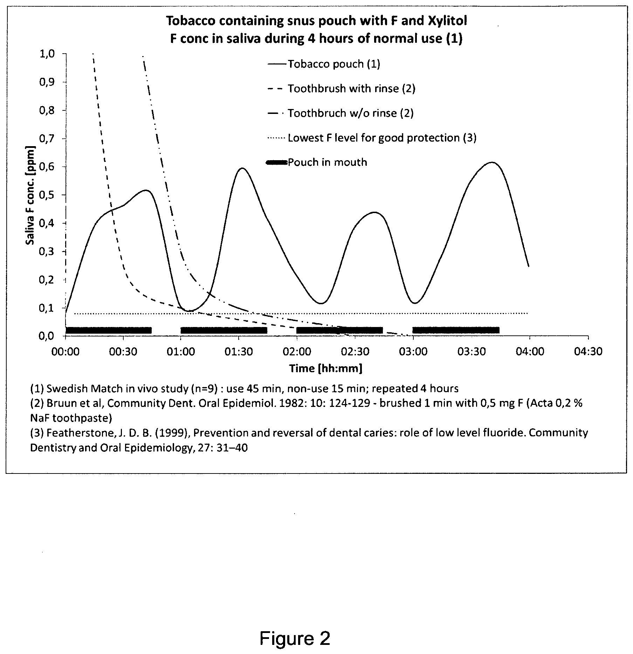

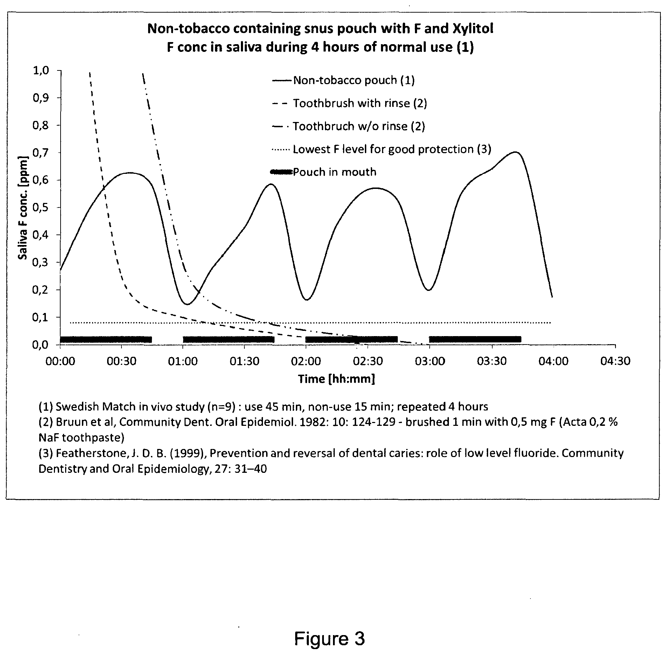

The present document also provides a product and a delivery means that sustains therapeutically effective levels of fluoride concentration in the mouth over the whole day, as shown in Figures 2 and 3.

The excellent inhibitory effect of the products of the present document on growth of mutans streptococci, i.e., their significant reduction in the oral cavity, has been proven in our *in vivo* studies as shown herein below.

In accordance with the present document, an oral smokeless tobacco product or an oral smokeless non-tobacco snuff product releasing xylitol and optionally also fluoride in the oral cavity is provided. Xylitol and optionally also fluoride may be incorporated in the matrix of the product, for example in the tobacco or non-tobacco plant material mixture of the product. The oral use of the product, normally between gum and lip, implies that the matrix slowly will be soaked by the saliva. Subsequently xylitol and fluoride if present will be released into the oral cavity. Thus, xylitol and also fluoride if present will gradually leave the product to be transported to the dental plaque by saliva passing through the product's matrix. The concentration of xylitol and fluoride will be the highest in areas close to where the product is placed, but the natural flow of saliva in the oral cavity will provide also distant dental sites with xylitol and fluoride. Hence, a xylitol and optionally fluoride releasing product with bacteria inhibiting properties is provided.

The long lasting tooth-protection, obtained in accordance with the present document, ensures a bacteria inhibiting effect during the entire period of time the product is used in the mouth, or a substantial part thereof. The described effect does not only protect the teeth from demineralization. The slow release of xylitol and fluoride (when present) and the distribution of xylitol and fluoride to distant sites in the oral cavity by the natural flow of saliva also underlie the long-lasting caries-preventive properties of the products. Hence, the product of the present document can be used for the purpose of protecting teeth from demineralization and caries lesions after e.g. the intake of a meal.

The product of the present document comprises water in an amount of 20-60 w/w %, such as 22-60 w/w %, 26-58 w/w%, 25-55 w/w %, 30-55 w/w %, 35-55 w/w %, 35-52 w/w%, or 44-52 w/w% of the final product as determined by Karl Fischer method or Gas Chromatography method.

The product of the present document may comprise humectants selected from the group of glycerol, propylene glycol or a combination thereof.

The product of the present document in addition to sodium chloride salt may also comprise additional salt(s), such as, sodium carbonate, calcium carbonate, sodium bicarbonate and/or magnesium carbonate and/or ammonium chloride. Such additional salt(s) may act as buffering and/or pH regulating agents. However, when fluoride is added in the product, sodium carbonate is preferred, since calcium may bind sodium fluoride.

Any known type of oral smokeless tobacco or oral non-tobacco snuff product may be used in accordance with the present document. The products may be shaped into any suitable form and packaged in any desirable type of packaging.

The oral smokeless tobacco may be e.g. moist snuff such as snus, or chewing tobacco, oral dry snuff or hard snuff. The oral smokeless non-tobacco snuff product may be shaped into a form resembling moist snuff, such as snus, or chewing tobacco, oral dry snuff or hard snuff.

The moist snuff product such as snus, or alternatively the oral smokeless non-tobacco snuff product shaped into a form resembling moist snuff, such as snus, may be in loose, particulate form, or pre-packed in portions such as pouches.

The chewing tobacco, or alternatively the oral smokeless non-tobacco product shaped into a form resembling chewing tobacco, may be in loose-leaf form, in the form of a plug, roll or twist, or as cut pieces of a strand.

The hard snuff or alternatively the oral smokeless non-tobacco product shaped into a form resembling hard snuff may be in the form of a film, strip, pellet, pod cake, stick, tablet or lozenge. Typically, the hard snuff does not comprise a gum base.

In one form a pouch of about 0.9 to 1 g may comprise about 6-20 w/w % of xylitol, such as about 9.0 to about 9.9 w/w % of xylitol and any number in between. In yet another form it may in addition comprise 0.0200-0.0250 w/w % (200-250 ppm) of fluoride.

The oral smokeless tobacco product or the oral smokeless non-tobacco snuff product may be packaged in a box, can, canister, cardboard box, bag, stick-pack wrapping, plastic wrapping, paper wrapping, foil wrapping, blister pack or on a tray.

The product comprising xylitol as defined herein may also be used as a delivery device for the delivery of xylitol and/or fluoride to the oral cavity. The present document is therefore also directed to the use of a product comprising xylitol according to the present document for delivering xylitol to the oral cavity.

Manufacturing processes of oral smokeless tobacco products, e.g. moist snuff such as snus, and chewing tobacco, are well known to the person skilled in the art, and any known process thereof may be used. Moist snuff is known as either Swedish-type snus or American-type moist snuff.

A general description of snus manufacturing is presented by e.g. ESTOC, European Smokeless Tobacco Council, and the GothiaTek® quality standard for snus. Methods for the manufacture of American type moist snuff and chewing tobacco are described in e.g. 'Wahlberg, I., Ringberger, T. (1999) Smokeless Tobacco. In: Tobacco: Production, Chemistry and Technology, (eds D.L. Davis & M.T. Nielsen) pp. 452-460. World Agriculture Series, Blackwell Science Ltd. Tobacco is the raw material in any oral smokeless tobacco product. However, for the reason of controlling the nicotine content of the products, the raw material may well be constituted of a mixture of tobacco and other plant materials.

The principle of snus manufacturing is to mix ground or cut tobacco with water and sodium chloride and heat treating the mixture for a period of time long enough (typically several hours), and at a temperature high enough, to meet the demands for pasteurization. The heat treatment also gives texture and colour to the mixture and enhances the natural tobacco flavours. After heat treatment the mixture is chilled. Additives such as pH-regulators and flavourings are then added and the mixture may be adjusted in moisture content. The ready-made blend is packed, typically in boxes as loose snus or as portions (pouches or sachets). Snus is typically used between the upper gum and lip and is well known as the Swedish-type of oral smokeless tobacco.

American-type moist snuff is commonly produced through a fermentation process of moisturized ground or cut tobacco. Flavours and ingredients are mixed to the blend and water is added to adjust the moisture content. American-type moist snuff is available in a loose form or as pre-packed pouches and is most commonly used between the lower gum and lip but could also be used as snus between the upper gum and lip.

Chewing tobacco is most often made of loose leaf tobacco, which is cured at a slightly elevated temperature. The tobacco leaves are then threshed into flakes and the mid-rids (stems) are removed. The tobacco fragments thus obtained are usually treated with a solution of flavours and additives, dried to lower the moisture content and packed in a consumer package. The product achieved is known as "loose-leaf chewing tobacco". The treated tobacco fragments could also be compressed to blocks of tobacco (product known as "plugs") or spun to thick strands of tobacco (product known as "twist"). For the Scandinavian type of chewing tobacco, the strands are thinner and cut into pieces. Chewing tobacco is normally used by putting a pinch of the loose leaf chewing tobacco or a bite of the plug or twist in the lower part of the mouth between the lower gum and lip. Scandinavian chewing tobacco, i.e. pieces of a strand, is normally used in the same way as snus. By chewing the tobacco once in a while, flavour is released more efficiently. Chewing tobacco as referred to here is the typical kind of chewing tobacco used in North America, commonly known as "chew" or "chaw", or Scandinavian chewing tobacco.

Hard snuff is a group of oral tobacco-based products intended for oral use as a delivery system of nicotine from tobacco. Besides the additive carrying the active substance, which is tobacco carrying nicotine, hard snuff products are generally constituted by entirely or substantially inert materials such as fibres and polymers. They may also be mainly constituted by powdered tobacco. Examples of hard snuff products are products in the shape of a film, strip, lozenge or tablet.

Dry oral snuff resembles snus and American-type moist snuff but is characterized by being made of a finely ground tobacco powder and having a low moisture content (typically less than 10%). The product may be heat-treated but is normally manufactured from fire-cured fermented tobacco which is ground into a powder to which other ingredients such as flavours are added.

Manufacturing of oral smokeless non-tobacco snuff products typically follows the procedure of manufacturing of oral smokeless tobacco products, adjusted to the non-tobacco material used, except for that tobacco is replaced by non-tobacco raw material, typically constituted of non-tobacco plant materials. Suitable plant materials are non-tobacco fibres, preferably characterized by having high dietary fibre content. Examples of such materials are oat fibres (dietary fibre content >85%), apple fibres (dietary fibre content approx. 50-60%), sugar beet fibres (dietary fibre content approx. 65-75%), potato fibres (dietary fibre content approx. 70%), corn fibres (dietary fibre content approx. 70-80%), buckwheat fibres (dietary fibre content approx. 90%), cocoa fibres (dietary fibre content approx. 50%), cellulose fibres (dietary fibre approx. 95-99%). Oral smokeless non-tobacco snuff products are used in the same manner as the corresponding oral smokeless tobacco products. They offer a healthier alternative to oral smokeless tobacco products, since they do not contain tobacco and most often do not contain any nicotine either. In accordance with the present document, oral smokeless non-tobacco snuff products may also be used for the administration of drugs, as delivery systems intended for oral use and controlled release of biologically active substances.

To produce the oral smokeless tobacco product or oral smokeless non-tobacco snuff product according to the present document, xylitol and optionally also fluoride may be added to the product mixture in any of the process steps of the manufacturing process. Thus, e.g. xylitol and/or fluoride may be added prior to or after the heat-treatment or fermentation step. The person skilled in the art would realize at what process step(s) xylitol and/or fluoride is/are most suitably added to the mixture. After addition of xylitol and optionally of fluoride, the tobacco or non-tobacco blend must be mixed enough to have the xylitol and fluoride (if fluoride is added) homogeneously distributed in the blend.

A product of the present document typically comprises about 25-60 w/w %, such as 27-60 w/w %, 25-55 w/w %, 30-50 w/w %, or 30-45 w/w % of dry tobacco or non-tobacco material (based on the product).

The xylitol and optionally the fluoride may also be added to the product in any other suitable manner of incorporating additives into smokeless products being known in the art.

The xylitol and optionally also fluoride containing tobacco or non-tobacco blend may be packed in loose, particulate form, loose-leaf form, in the form of a pre-packed portion such as a pouch or sachet, in the form of a pellet, pod, cake, film, strip, tablet, lozenge or stick, as pieces of a strand or in the form of a plug or twist. The ready-made product may be packed in a consumer package such as a box, can, canister, cardboard box, bag, stick-pack wrapping, plastic wrapping, paper wrapping, foil wrapping, blister pack or on a tray.

### Method of evaluation

In order to prove the effect of regular use of the product of the present document, which comprises a relatively low amount of xylitol, on the content of mutans streptococci in the mouth, a series of clinical studies were performed as set out herein below.

### Recruiting

Snus users were screened for mouth content of mutans streptococci (MS). To be included in the study, the persons should use at least 10 pouches of snus a day, not use antibiotics, have most of their regular teeth left and have a MS count above 95 000 CFU/ml saliva. 10 persons fulfilling the above requirements were included in the study: 6 males and 4 females between 23 and 56 years of age.

### Restrictions

The participants were asked to stop using any other xylitol containing product during the study. Tooth brushing was made with a regular standardized tooth paste which was provided to them. The participants were not allowed to eat or drink anything or use any tobacco product 2 hours before saliva sampling. They were asked to use the provided snus as they would normally use their usual snus.

### Sampling and measurement of mutans streptococci in the saliva

Number of mutans streptococci (MS) in the saliva was measured at baseline and after 4 weeks for all participants.

The participants were asked to spit in a sample vial during 4 minutes, while chewing on a piece of paraffin to stimulate the saliva production (paraffin stimulates whole saliva). One ml of the obtained saliva was transferred to a vial filled with a transport fluid and sent to an external laboratory for analysis of MS. In the laboratory the samples were diluted and put on mitis salivarius Agar plates. After incubation the number of Colony Forming Units (CFU) were counted. The result was expressed as CFU per milliliter (CFU/ml) saliva. For details about the analysis method reference is made to Jannesson et al., Caries Res. 2002 Jan-Feb;36(1):36-9.

### Xylitol analysis

Samples were collected from the mouth (saliva), for direct measurement of xylitol concentration in saliva (cf. example 4). For measurement of the remaining xylitol amount in the used product (cf. example 3), the used products were collected and extracted in Milli-Q water prior to further treatment. All samples were then filtrated through a filter with a pore size of less than 0.45 µm. The filtrates were analysed by HPLC with a Sugar-Pak I column and a RI-detector. A new calibration curve was made for each analysis time. The content calculations were done with Agilent Chemstation and Wilab-LIMS.

### Fluoride (F) analysis

Saliva samples were collected as set out in example 6. 200 µl of the collected saliva sample was mixed with 20 µl of Total Ionic Strength Adjustment Buffer (TISAB III, Thermo Electron Corp., Waltham, MA, USA). 100 µl of the solution was placed as a drop on a Petri dish, and the F concentration was measured by carefully lowering an ion-specific electrode (Model 96-09, Orion Research Inc., Fisher Scientific, Loughborough, UK) into the fluid. The surface tension of the drop ensured that the liquid covered the entire membrane surface of the electrode. In order to calibrate the electrode, three standard solutions were used (comprising 0.1, 1, and 10 ppm F respectively). The F concentration in saliva was expressed as parts per million (ppm).

In case used snus pouches were analysed, they were first extracted in 100ml Milli-Q water/pouch at room temperature for three hours with agitation and afterwards the same procedure as that for the saliva samples described above was followed.

### Examples

### Example 1: Portion packed tobacco snus product containing xylitol and fluoride (F)

20 kg snus was manufactured in accordance with the GothiaTek® standard.

A mixture of ground tobacco, sodium chloride, non-tobacco bamboo fiber BAF400DV (in accordance with patent application WO 2013/152918) and water was heat-treated at a temperature of 100°C for 2 hours and thereafter at a declining temperature from 100°C to 70°C for 8 hours. The mixture was then chilled to room temperature.

Additional components were added to the chilled blend; flavourings for a pleasant aroma and taste; water for moisture adjustment; Xylitol (DuPont-Denison Xavier C or CM grade) and Sodium fluoride (purity >99%, ACS reagent powder, Acros Organics, Geel, Belgium), were added after dissolution in water. The blend was then further mixed.

The snus-blend was packed to portions using a SM NYPS machine (US Patent Specification No. 6.135.120, "Device for packing of finely divided, moistened tobacco material", Löfman et al.). Standard viscose based non-woven fabric for snus was used as wrapping material for the snus portions. Each pouch, including pouch paper, had a weight of 0.9 g and a composition according to Table 1.

**Table 1**

| **Ingredient** | **% w/w** |
|---|---|
| Tobacco | 29.5% |
| Sodium chloride | 3.3% |
| Bamboo fiber (BAF 400DV) | 7.6% |
| Water | 43.9% |
| Sodium carbonate | 1.7% |
| Xylitol | 9.4%* |
| Sodium fluoride** | 0.049% |
| Flavours | 0.37% |
| Pouch paper | 4.2% |
| Total | 100% |

| | |
|---|---|
| *9.4% as analysed based on the pouch with paper. The recipe for the pure snus was 9.9% and this sample will be referred to as 9.9% xylitol in following examples **corresponding to 0.022 % w/w F | |

### Example 2: A portion packed non-tobacco snus product containing fluoride (F)

Non-tobacco snus pouches were made following the manufacture procedure of Example 1. The obtained pouches, including pouch paper, had each a weight of 0.9 g and a composition according to Table 2.

**Table 2**

| Ingredient | % w/w |
|---|---|
| Oat and cocoa fiber mix | 39.2% |
| Sodium chloride | 5.3% |
| Glycerol | 5.4% |
| water | 42.3% |
| Sodium carbonate | 0.72% |
| Magnesium carbonate | 1.0% |
| Sodium fluoride* | 0.044% |
| Flavours | 1.7% |
| Pouch paper | 4.2% |
| Total | 100.00% |

| | |
|---|---|
| *corresponding to 0.020 % w/w F | |

### Example 3: calculation of xylitol concentration in saliva based on measurements of remaining xylitol in the used products

Five persons used snus pouches manufactured according to Example 1.

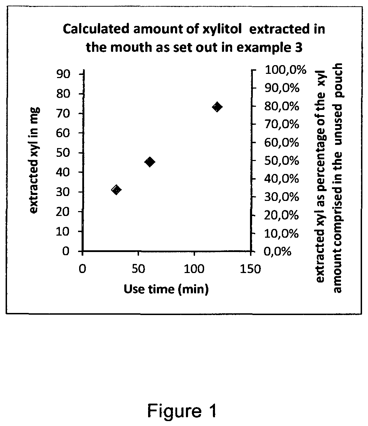

The persons were instructed to use snus pouches for 30, 60 and 120 minutes respectively, without eating or drinking in between. The used pouches were collected in sample tubes and analysed for remaining xylitol content. The analysis of xylitol was performed according to that described herein above under Xylitol analysis. Based on the measured amount of remaining xylitol in the used pouch and the known amount of xylitol comprised in the unused product, the amount of xylitol extracted into the mouth was calculated, expressed both as a net weight in mg and as a percentage of the xylitol amount originally comprised in the unused product. The obtained results are shown in Figure 1.

As can be seen from Figure 1, the release of xylitol from the pouch into the mouth continues over two hours, if a pouch is used for that long. During the first 30 min of use, the average release rate of xylitol was 1.0 mg/min. After 30 min, 60 min, or up to 120 min of use, the release rate of xylitol was 0.47mg/min. This even release of xylitol over time ensures a steady concentration of xylitol in the mouth.

An unstimulated whole saliva flow rate in a normal person is 0.3-0.4 ml per minute [Coulthard [et al.], Paul (2008), Oral and Maxillofacial Surgery, Radiology, Pathology and Oral Medicine (2nd ed.), Edinburgh: Churchill Livingstone/Elsevier, pp. 210, 212-213, (ISBN 9780443068966). Thus, if counting on a mean salivary rate of 0.35 ml/min and a saliva density equal to that of water and using the above-calculated flow rates of xylitol release in the mouth in relation to the use time, the steady-state xylitol concentrations in the saliva shown in Table 3 could be calculated.

**Table 3**

| Use time interval | Calculated xylitol concentration in saliva (%) |
|---|---|
| 0-30 min | 0.29% |
| 30-120 min | 0.13% |

### Example 4: In-vivo measurement of xylitol concentration in the saliva.

Xylitol containing snus pouches made according to Example 1 were used and unstimulated whole saliva samples were collected after 15 and 30 min respectively. The analysis of xylitol was performed according to that described herein above under Xylitol analysis. The average measured xylitol concentration in the saliva is shown in Table 4.

**Table 4**

| Sample time | Xylitol in saliva (%) |
|---|---|
| 15min | 0.27% |
| 30 min | 0.16% |

As can be seen, the values shown in Table 4 are similar to those shown in Table 3 above, thus the calculated values of xylitol concentration in the saliva are in compliance with those measured directly from the saliva.

In Holgerson et al., Caries Res 2006; 40:393-397, the effect on xylitol concentration in the saliva by using normal xylitol containing products is presented. The highest concentration was obtained from use of chewing gum with 1.3 g xylitol per piece: 3.37% xylitol in the saliva The lowest effect was obtained from use of a toothpaste containing 0.1 g of xylitol per dose: 0.82% xylitol in the saliva.

Thus, according to the above studies, the xylitol concentration in the saliva obtained by using snus pouches comprising xylitol according to the present document is significantly lower than that obtained from use of commercially available xylitol comprising products.

### Example 5 - Clinical test of decrease of Mutans Streptococci

Tobacco containing snus manufactured according to Example 1 was used in this study. 10 persons recruited as stated above under method of evaluation were included in the study. Sampling and analysis was performed as stated herein above under method of evaluation.

After 4 weeks 9 out of the 10 participants had reduced MS levels. In average per participant, the reduction was 54% compared to baseline levels. (The average logarithmic {log [MS]0w - log [MS]4w} decrease was 0.59).

Discussion: The average available daily dosage of xylitol provided to the participants in the study was 2.6 g (0.0842 g/pouch) per day (depending on the number of snus pouches used). Taking then into consideration that less than 50% of the available xylitol was extracted from the pouches during a normal usage time of about 60 min or less (cf. Example 3), the real active daily dosage of xylitol extracted in the mouth was less than 1.3 g.

In the study presented by Milgrom et al., J Dent Res. 2006 February; 85(2): 177-181, xylitol tablets were taken three times a day, the minimum dosage for a reductive effect on Mutans Streptococci was found to be 3.44 g per day (1.15 g/piece chewing gum). Thus, as shown above, the products of the present document even though providing for a much lower daily dosage of xylitol compared to other known oral products containing xylitol, they provided for a much better reductive effect on mutans streptococci compared to other known oral products comprising xylitol.

### Example 6: Effect of fluoride containing snus on fluoride concentration in the saliva

9 persons participated in the study and snus pouches manufactured according to Example 1 were used.

In order to simulate a normal moderate snus usage, the participants used a pouch for 45 min and then a 15 min break was taken before placing the next pouch in the mouth. This cycle was then repeated three times - in total 4 pouches were used during a 4 hours period. The participants were not allowed to drink or eat anything during the study. Samples of unstimulated whole saliva were collected at 0 min and then every 15 minutes during 4 hours. Analysis of the fluoride concentration in the saliva was performed as stated herein above under fluoride analysis.

Results: The results from the study are depicted in Figure 2, together with reference values of fluoride concentrations in the saliva after tooth brushing with and without rinsing [Bruun et al., Community Dent. Oral Epidemiol. 1982: 10: 124-129]. The lowest level of fluoride concentration in the saliva for a maximum caries protection according to Featherstone, J. D. B. (1999), Prevention and reversal of dental caries: role of low level fluoride. Community Dentistry and Oral Epidemiology, 27: 31-40, is also shown in Figure 2.

Discussion: As outlined in Featherstone, J. D. B. (1999), Prevention and reversal of dental caries: role of low level fluoride. Community Dentistry and Oral Epidemiology, 27: 31-40, the caries preventing action of fluoride is due to several mechanisms and not only due to the long term enamel strengthening effect. One implication is that it is favourable to have a certain background concentration of fluoride present in the saliva, especially when an acid attack occurs in connection with meals and drinking. Further, maximum prevention is achieved if the background concentration of fluoride present in the saliva is 0.08 ppm or higher.

As can be seen from Figure 2, the fluoride concentration in the saliva never drops below the background concentration of fluoride in the saliva, as determined according to Featherstone publication mentioned above, during moderate use of the fluoride containing snus pouches according to the present document. Thus, moderate use of a product comprising fluoride according to the present document provides for maintaining an effective fluoride concentration in the mouth throughout the whole day. This effect cannot be obtained by only using a toothpaste comprising fluoride as shown in Figure 2, after tooth brushing, the fluoride concentration in the saliva drops below the effective level already after 1h 15 min (with rinsing after brushing) and 1h 45 min (without rinsing after brushing).

### Example 7: Effect of fluoride containing non-tobacco snus on fluoride concentration in the saliva

8 persons participated in the study and snus pouches manufactured according to Example 2 were used. The study was conducted in the same way as that according to Example 6.

Results: The results from this study are depicted in Figure 3, where they are compared to the same reference values of fluoride concentration in the saliva as those shown in Figure 2.

Discussion: Use of non-tobacco pouches comprising fluoride according to the present document provides for as good effect as that obtained when tobacco containing pouches according to example 6 above were used.

### Example 8: Effect of xylitol containing snus on xylitol concentration in the saliva

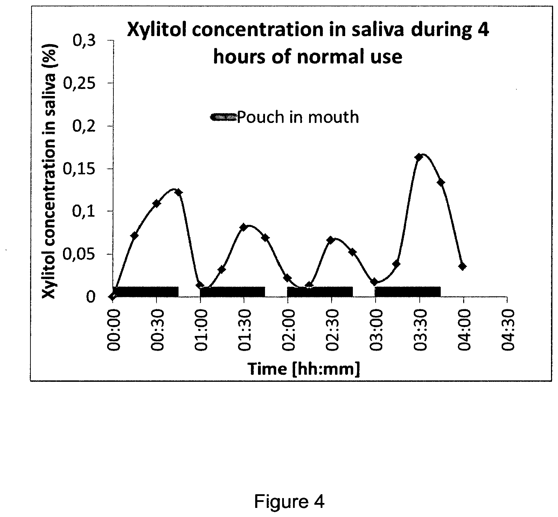

9 persons participated in the study and snus pouches manufactured according to Example 1 were used. In order to simulate a normal moderate snus usage, the participants used a pouch for 45 min and then a 15 min break was taken before placing the next pouch in the mouth. This cycle was then repeated three times - in total 4 pouches were used during a 4 hours period. The participants were not allowed to drink or eat anything during the study. Samples of unstimulated whole saliva were collected at 0 min and then every 15 minutes during 4 hours. Analysis of the xylitol concentration in the saliva was performed as stated herein above under xylitol analysis. The results from the study are depicted in Figure 4.

The variation in area under the peaks for the four 60 min periods can be attributed to normal variation of salivary flow and different placements of the pouch under the lip. The average saliva xylitol concentrations during the four 60 min periods are presented in Table 5. Thus, the product allows the release of xylitol to obtain an average concentration of xylitol in saliva well above 0.02%, and even above 0.03%, during normal use of the product.

**Table 5**

| Period | Average xylitol in saliva (%) |
|---|---|
| 1 | 0.054 |
| 2 | 0.043 |
| 3 | 0.034 |
| 4 | 0.077 |

### Example 9: Portion packed tobacco snus product containing xylitol at different levels

A snus-material was manufactured in accordance with the GothiaTek® standard. A mixture of ground tobacco, sodium chloride, propylene glycol and water was heat-treated at a temperature of 100°C for 2 hours and thereafter at a declining temperature from 100°C to 70°C for 8 hours. Sodium carbonate was then added for pH adjustment and the mixture was then chilled to room temperature.

Additional components were added to the chilled blend; extra sodium chloride, flavourings and sweetener (acesulfam K) for a pleasant aroma and taste; water for moisture adjustment; Xylitol (DuPont-Denison Xavier C or CM grade) and Sodium fluoride (purity >99%, ACS reagent powder, Acros Organics, Geel, Belgium). The blend was then further mixed.

The snus-blends were packed to portions using a standard stick-pack machine (Merz). Standard viscose based non-woven fabric for snus was used as wrapping material for the snus portions. Each pouch used for further experiments, including pouch paper, had a weight of 0.65±0.05g. The pouch paper represented 5.8% of the final weight.

Three samples were made with a snus composition (excluding pouch paper) according to Table 6.

**Table 6**

| **Ingredient** | **S1 (% w/w)** | **S2 (% w/w)** | **S3 (% w/w)** |
|---|---|---|---|
| Tobacco | 51.4% | 46.9% | 42.5% |
| Sodium chloride | 2.0% | 1.8% | 1.6% |
| Propylene glycol | 2.9% | 2.7% | 2.4% |
| Sodium carbonate | 2.7% | 2.4% | 2.2% |
| Sodium fluoride* | 0.046% | 0.042% | 0.038% |
| Acesulfam K | 0.059% | 0.054% | 0.049% |
| Xylitol | 5.2% | 10.3% | 15.4% |
| Flavours | 2.7% | 2.4% | 2.2% |
| water | 33.1% | 33.3% | 33.5% |
| **SUM** | **100%** | **100%** | **100%** |
| | | | |
| *Pure F of NaF added | 0.0209% | 0.0191% | 0.0173% |

### Example 10 - Relation between xylitol concentration in snus and saliva

Snus pouches from Example 9: S1, S2 and S3 with 5.2%, 10.3% and 15.4% xylitol, were used to measure the relationship between xylitol concentration in the snus and the resulting xylitol concentration in saliva. The analysis of xylitol was performed according to that described herein above under Xylitol analysis.

Each snus type, S1, S2 and S3, were used by 5 to 9 persons. Before putting the snus pouch in the mouth, they were instructed to spit in a sample vial, giving a baseline sample. After 15 min snus usage they were instructed to provide another saliva sample. The saliva samples for the same snus type (S1-S3) and time (baseline and 15min) were pooled prior to analysis.
The participants had not used any xylitol containing products the same day as the test and all the baseline samples were below detection limit.

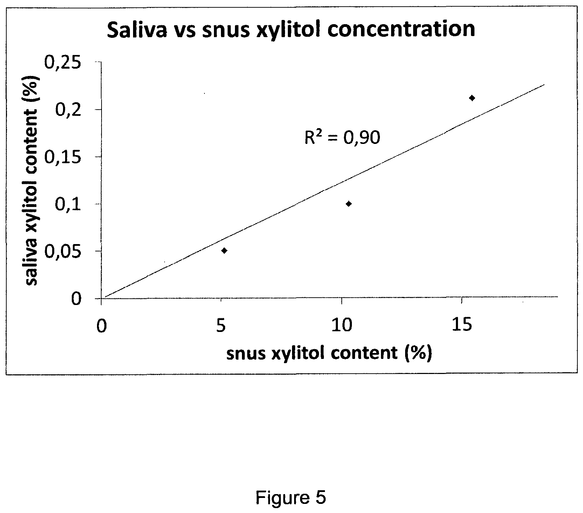

The relationship between saliva and snus concentration of xylitol is depicted in Figure 5. As seen, the saliva xylitol concentration is relatively linearly dependent on the snus xylitol concentration.

### Example 11 - Xylitol delivery during prolonged usage times

In Example 8, a typical normal snus usage was simulated. The present example demonstrates that a steady state delivery of xylitol can be achieved even if a person uses the pouch for a longer period than 45 min.
Two persons used S2 pouches from experiment 9 with 10.3% xylitol content of the snus. The persons had not used any xylitol containing products the same day as the experiment. Before putting the snus pouch in the mouth they gave a 0 min baseline sample by spitting in a sample vial. During usage they provided saliva samples after 30, 60, 90 and 120 min. The samples for each time were pooled prior to analysis. The analysis of xylitol was performed according to that described herein above under Xylitol analysis.

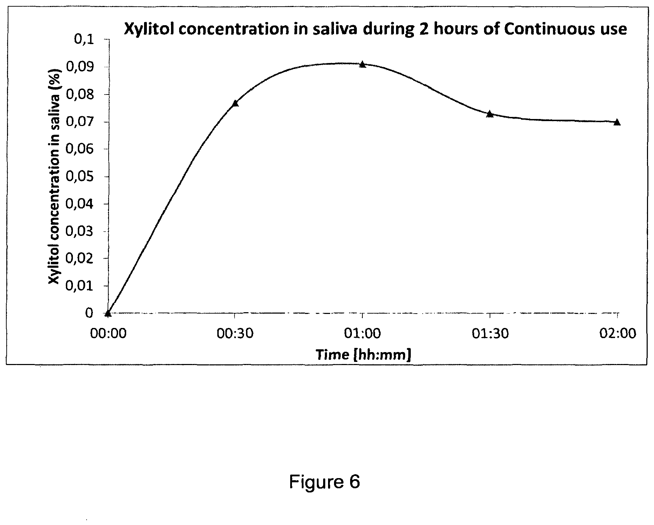

The result is depicted in Figure 6. As can be seen, there still is a steady delivery of xylitol even after 2h of usage.

Various embodiments of the present document have been described above, but a person skilled in the art realizes further minor alterations, which would fall into the scope of the present document. The breadth and scope of the present document should not be limited by any of the above-described exemplary embodiments, but should be defined only in accordance with the following claims and their equivalents. Other aspects, advantages and modifications within the scope of the present document will be apparent to those skilled in the art to which the present document pertains.

## Claims

1. An oral smokeless tobacco product or an oral smokeless non-tobacco snuff product, comprising xylitol in an amount of from 6 to 20 w/w % of the final product, wherein the water content of the final product is 20 to 60 w/w %, and wherein said oral smokeless non-tobacco snuff product comprises non-tobacco plant material, wherein said non-tobacco plant material is non-tobacco fibers.

2. A product according to claim 1, wherein the amount of xylitol is from 6 to 15 w/w % of the final product.

3. A product according to claim 1 or 2, wherein the amount of xylitol is from 9 to 9.9 % of the final product.

4. A product according to any one of the preceding claims, wherein the water content of the final product is 26 to 58 w/w %.

5. A product according to any one of the preceding claims, wherein it further comprises fluoride added as sodium fluoride and/or sodium monofluorophosphate.

6. A product according to claim 5, wherein the fluoride is added as sodium fluoride.

7. A product according to any one of the preceding claims, wherein the fluoride is present in an amount of from 0.0050 to 0.0500 w/w % of the final product, such as from 0.0150 to 0.0300 w/w % of the final product.

8. A product according to any one of the preceding claims, wherein the non-tobacco fibers are oat fibres, apple fibres, sugar beet fibres, potato fibres, corn fibres, buckwheat fibres, cocoa fibres, and/or cellulose fibres.

9. A product according to any one of the preceding claims, wherein the oral smokeless tobacco product is moist snuff such as snus, or chewing tobacco.

10. A product according to any one of claims 1-8, wherein the oral smokeless non-tobacco snuff product is shaped into a form resembling moist snuff such as snus, or chewing tobacco, oral dry snuff or hard snuff.

11. A product according to any one of the preceding claims, wherein the product is provided in form of pouches, or loose in particulate form, packed in cans or boxes.

12. A product according to claim 11, wherein the product is provided in form of pouches.

13. An oral smokeless tobacco product or an oral smokeless non-tobacco snuff product as defined in any one of claims 1-2 for use in the treatment and/or prevention of periodontal disease and/or dental caries.

## Patentansprüche

1. Orales rauchfreies Tabakprodukt oder ein orales rauchfreies Nichttabak-Schnupfprodukt, umfassend Xylit in einer Menge von 6 bis 20% Gew./Gew. des Endprodukts, wobei der Wassergehalt des Endprodukts 20 bis 60 % Gew./Gew. beträgt und wobei das orale rauchfreie Nichttabak-Schnupfprodukt Nichttabak-Pflanzenmaterial umfasst, wobei das Nichttabak-Pflanzenmaterial Nichttabakfasern ist.

2. Produkt nach Anspruch 1, wobei die Menge an Xylit 6 bis 15 % Gew./Gew. des Endprodukts beträgt.

3. Produkt nach Anspruch 1 oder 2, wobei die Menge an Xylit 9 bis 9,9 % des Endprodukts beträgt.

4. Produkt nach einem der vorstehenden Ansprüche, wobei der Wassergehalt des Endprodukts 26 bis 58 % Gew./Gew. beträgt.

5. Produkt nach einem der vorstehenden Ansprüche, wobei es weiter Fluorid umfasst, das als Natriumfluorid und/oder Natriummonofluorophosphat zugesetzt wird.

6. Produkt nach Anspruch 5, wobei das Fluorid als Natriumfluorid zugegeben wird.

7. Produkt nach einem der vorstehenden Ansprüche, wobei das Fluorid in einer Menge von 0,0050 bis 0,0500 % Gew./Gew. des Endprodukts, wie beispielsweise 0,0150 bis 0,0300 % Gew./Gew. des Endprodukts, vorhanden ist.

8. Produkt nach einem der vorstehenden Ansprüche, wobei die Nichttabakfasern Haferfasern, Apfelfasern, Zuckerrübenfasern, Kartoffelfasern, Maisfasern, Buchweizenfasern, Kakaofasern und/oder Cellulosefasern sind.

9. Produkt nach einem der vorstehenden Ansprüche, wobei das orale rauchfreie Tabakprodukt feuchter Schnupftabak, wie Snus, oder Kautabak ist.

10. Produkt nach einem der Ansprüche 1-8, wobei das orale rauchfreie Nichttabak-Schnupfprodukt zu einer Form geformt ist, die feuchtem Schnupftabak, wie Snus, oder Kautabak, oralem trockenem Schnupftabak oder hartem Schnupftabak ähnelt.

11. Produkt nach einem der vorstehenden Ansprüche, wobei das Produkt in Form von Beuteln oder lose in Teilchenform, verpackt in Dosen oder Kartons, bereitgestellt wird.

12. Produkt nach Anspruch 11, wobei das Produkt in Form von Beuteln bereitgestellt wird.

13. Orales rauchfreies Tabakprodukt oder ein orales rauchfreies Nicht-Tabak-Schnupfprodukt nach einem der Ansprüche 1-2 zur Verwendung bei der Behandlung und/oder Prävention von Parodontalerkrankungen und/oder Zahnkaries.

## Revendications

1. Produit de tabac sans fumée à usage oral ou produit à priser sans tabac et sans fumée à usage oral, comprenant du xylitol en une quantité de 6 à 20 % en poids du produit final, dans lequel la teneur en eau du produit final est de 20 à 60 % en poids, et dans lequel ledit produit à priser sans tabac et sans fumée à usage oral comprend une matière végétale sans tabac, dans lequel ladite matière végétale sans tabac est faite de fibres autres que des fibres de tabac.

2. Produit selon la revendication 1, dans lequel la quantité de xylitol va de 6 à 15 % en poids du produit final.

3. Produit selon la revendication 1 ou 2, dans lequel la quantité de xylitol va de 9 à 9,9 % en poids du produit final.

4. Produit selon l'une quelconque des revendications précédentes, dans lequel la teneur en eau du produit final va de 26 à 58 % en poids.

5. Produit selon l'une quelconque des revendications précédentes, dans lequel ledit produit comprend en outre du fluorure ajouté sous forme de fluorure de sodium et/ou de monofluorophosphate de sodium.

6. Produit selon la revendication 5, dans lequel le fluorure est ajouté sous forme de fluorure de sodium.

7. Produit selon l'une quelconque des revendications précédentes, dans lequel le fluorure est présent en une quantité allant de 0,0050 à 0,0500 % en poids du produit final, par exemple de 0,0150 à 0,0300 % en poids du produit final.

8. Produit selon l'une quelconque des revendications précédentes, dans lequel les fibres autres que des fibres de tabac sont des fibres d'avoine, des fibres de pomme, des fibres de betterave à sucre, des fibres de pomme de terre, des fibres de maïs, des fibres de sarrasin, des fibres de cacao et/ou des fibres de cellulose.

9. Produit selon l'une quelconque des revendications précédentes, dans lequel le produit de tabac sans fumée à usage oral est un tabac à priser humide tel que le snus, ou un tabac à chiquer.

10. Produit selon l'une quelconque des revendications 1 à 8, dans lequel le produit à priser sans tabac et sans fumée à usage oral se présente sous une forme ressemblant à celle d'un tabac à priser humide tel que le snus, ou d'un tabac à chiquer, d'un tabac à priser sec à usage orale ou d'un tabac à priser dur.

11. Produit selon l'une quelconque des revendications précédentes, dans lequel le produit est fourni sous forme de sachets, ou en vrac sous forme particulaire, emballé dans des boîtes ou des pots.

12. Produit selon la revendication 11, dans lequel le produit est fourni sous forme de sachets.

13. Produit de tabac sans fumée à usage oral ou produit à priser sans tabac et sans fumée à usage oral selon l'une quelconque des revendications 1 et 2 destiné à être utilisé dans le traitement et/ou la prévention de la maladie parodontale et/ou des caries dentaires.
